# EUROPEAN PATENT SPECIFICATION

(11) **EP 3 942 036 B1**
(45) Date of publication and mention of the grant of the patent: **31.05.2023**
(21) Application number: 20719050.5
(22) Date of filing: 20.03.2020
(51) Int. Cl.: C12N 15/09, C12N 15/10, C12N 15/11, C12Q 1/68, C12Q 1/6816, C12Q 1/6811, C12Q 1/6855, C12Q 1/682, C12Q 1/6862, C12Q 1/6865, C12Q 1/6897

(54) **SYSTEM**
SYSTEM
SYSTÈME

(30) Priority: 21.03.2019 US 201962821877 P; 10.02.2020 US 202062972599 P
(43) Date of publication of application: 26.01.2022
(73) Proprietor: Sherlock Biosciences, Inc., Watertown, MA 02472 (US)
(72) Inventor: BROWN III, Carl Wayne, Watertown, MA 02472 (US); BLAKE, William J., Watertown, MA 02472 (US); DHANDA, Rahul K., Watertown, MA 02472 (US)
(74) Representative: HGF
(86) International application number: PCT/US2020/024055
(87) International publication number: WO 2020/191376

(56) References cited:
- WO-A1-2018/107129
- WO-A1-2020/124050
- KAIXIANG ZHANG ET AL: "Direct Visualization of Single-Nucleotide Variation in mtDNA Using a CRISPR/Cas9-Mediated Proximity Ligation Assay", JOURNAL OF THE AMERICAN CHEMICAL SOCIETY, vol. 140, no. 36, 12 September 2018 (2018-09-12), pages 11293-11301, XP055552112, US ISSN: 0002-7863, DOI: 10.1021/jacs.8b05309

## Description

### Background

It is increasingly important that technologies be developed that can specifically detect nucleic acids of interest, particularly when present in very low abundance.

### Summary

The present disclosure describes technologies that permit sensitive detection of nucleic acids of interest (i.e., nucleic acids whose nucleotide sequence is or includes a target sequence). The present invention is set out in the claims.

In some embodiments, provided technologies utilize nucleic acid cleavage activity characteristic of certain Cas enzymes; in many embodiments, provided technologies utilize collateral cleavage activity of certain Cas enzymes. The present disclosure identifies the source of a problem associated with certain previously-developed technologies that utilize Cas collateral cleavage activity. The present disclosure solves such problems and, moreover, provides technologies with unexpected benefits and/or capabilities relative to alternative available systems.

Importantly, in many embodiments, the present disclosure provides technologies that de-couple sequence detection from activation of cleavage by a Cas enzyme. The present disclosure therefore represents a significant departure from most Cas-based technology systems, which focus on the hallmark of Cas enzymes: that their activity can be directed specifically to any sequence of interest via manipulation of guide RNA sequence.

In some embodiments, the present disclosure provides insights that certain advantages can be achieved and/or problems can be solved by taking the opposite approach - using a Cas system to detect a particular sequence but without engineering the Cas guide RNA to hybridize with that sequence. In some embodiments, the present disclosure teaches the use of a ligation-based system for initial target nucleic acid hybridization, where components of this ligation-based system are designed so that the ligation event generates nucleic acid molecule(s) that is/are, or that template, activating nucleic acids for Cas collateral cleavage activity. Provided systems permit the activating nucleic acids to have a sequence that need not be found in the original target nucleic acid. In some embodiments, therefore, the present disclosure provides technologies that can utilize one or a relatively small number of guide RNA sequences to detect a variety of different nucleic acid targets of interest, including targets that differ by a single base from other nucleic acid sequences.

Alternatively or additionally, in some embodiments, the present disclosure provides strategies that permit nucleic acid detection, even with dramatic (e.g., attomolar) sensitivity, without amplification of the initial target nucleic acid.

Advantages of provided technologies include, among other things, that (i) pre-amplification of target nucleic acid can be eliminated, thereby removing a potential source of sequence mutation that could distort assay results (e.g., via generating false positive and/or false negative read-outs); (ii) de-coupling target detection from activation of Cas collateral activity avoids the need to specifically design a different gRNA for each target sequence of interest; (iii) de-coupling target detection from activation of Cas collateral activity furthermore permits flexibility in selection of Cas system, as either or both of DNA and RNA can be generated and/or utilized activating nucleic acids; (iv) ligation specificity enables single base discrimination; (v) de-coupling target detection from activation of Cas collateral activity permits, among other things, multimerization of Cas activation sequences (i.e., those bound by a gRNA/crRNA), thereby potentially permitting activation of multiple Cas enzymes from a single ligation product (or template or copy thereof); and (vi) combinations thereof.

### Brief Description of the Drawing

*Figures 1A* and *1B**,* together, depict an exemplary embodiment of nucleic acid detection in accordance with the present disclosure.
*Figure 2* depicts an exemplary embodiment of nucleic acid detection in accordance with the present disclosure.

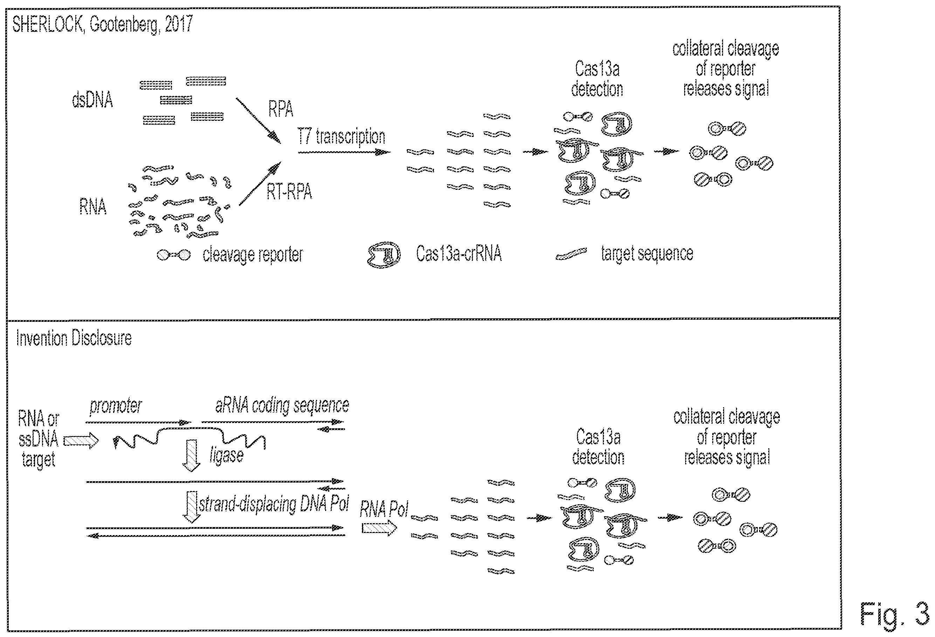

*Figure 3* presents a comparison of a SHERLOCK^{™} process (top panel, from Science 356:438, 2017) and technology as described herein for detection of nucleic acid using ligation-based transcriptional activation and CRISPR collateral activity (bottom panel).
*Figure 4* depicts an exemplary embodiment of increases to rate and/or amount of aRNA produced.
*Figure 5* depicts an exemplary embodiment of a multiplexed analysis as described herein.
*Figure 6* depicts an exemplary embodiment of a multiplexed analysis as described herein, including illustration of how such an analysis may be implemented, for example, via a fluidic system such as, for example, a honeycomb system (e.g., as is described in US Patent Application US2014/0087958 to Cepheid.).
*Figure 7* depicts exemplary ligation oligonucleotide sets with varied placement of the Cas recognition element. Hyb-Cas: Hybridization region upstream of the Cas target region, T7 Promoter and Cas target on separate strands; Cas-Hyb: Hybridization region downstream of the Cas target region, T7 Promoter and Cas target on the same strand.
*Figure 8* depicts different reporter types (RNA, dsDNA transcribed by RNA polymerase, and ssDNA converted to dsDNA *in situ*) tested to ensure efficient production of RNA from ssDNA sensors.
*Figure 9* demonstrates detection by Cas13 of RNA reporters generated from sequences described in Figure 8.
*Figures 10A* *and* *10B* demonstrate successful target RNA detection when the target hybridization region is placed upstream or downstream of the Cas target sequence.
*Figures 11A and 11B* demonstrate activation of *Lwa* Cas13 activity by single-stranded DNA (ssDNA).

### Definitions

***Amino acid:*** in its broadest sense, as used herein, refers to any compound and/or substance that can be incorporated into a polypeptide chain, e.g., through formation of one or more peptide bonds. In some embodiments, an amino acid has the general structure H₂N-C(H)(R)-COOH. In some embodiments, an amino acid is a naturally-occurring amino acid. In some embodiments, an amino acid is a non-natural amino acid; in some embodiments, an amino acid is a D-amino acid; in some embodiments, an amino acid is an L-amino acid. "Standard amino acid" refers to any of the twenty standard L-amino acids commonly found in naturally occurring peptides. "Nonstandard amino acid" refers to any amino acid, other than the standard amino acids, regardless of whether it is prepared synthetically or obtained from a natural source. In some embodiments, an amino acid, including a carboxy- and/or amino-terminal amino acid in a polypeptide, can contain a structural modification as compared with the general structure above. For example, in some embodiments, an amino acid may be modified by methylation, amidation, acetylation, pegylation, glycosylation, phosphorylation, and/or substitution (e.g., of the amino group, the carboxylic acid group, one or more protons, and/or the hydroxyl group) as compared with the general structure. In some embodiments, such modification may, for example, alter the circulating half-life of a polypeptide containing the modified amino acid as compared with one containing an otherwise identical unmodified amino acid. In some embodiments, such modification does not significantly alter a relevant activity of a polypeptide containing the modified amino acid, as compared with one containing an otherwise identical unmodified amino acid. As will be clear from context, in some embodiments, the term "amino acid" may be used to refer to a free amino acid; in some embodiments it may be used to refer to an amino acid residue of a polypeptide.

***Analog:*** As used herein, the term "analog" refers to a substance that shares one or more particular structural features, elements, components, or moieties with a reference substance. Typically, an "analog" shows significant structural similarity with the reference substance, for example sharing a core or consensus structure, but also differs in certain discrete ways. In some embodiments, an analog is a substance that can be generated from the reference substance, e.g., by chemical manipulation of the reference substance. In some embodiments, an analog is a substance that can be generated through performance of a synthetic process substantially similar to (e.g., sharing a plurality of steps with) one that generates the reference substance. In some embodiments, an analog is or can be generated through performance of a synthetic process different from that used to generate the reference substance.

***Associated:*** Two events or entities are "associated" with one another, as that term is used herein, if the presence, level, degree, type and/or form of one is correlated with that of the other. For example, a particular entity (e.g., polypeptide, genetic signature, metabolite, microbe, etc) is considered to be associated with a particular disease, disorder, or condition, if its presence, level and/or form correlates with incidence of and/or susceptibility to the disease, disorder, or condition (e.g., across a relevant population). In some embodiments, two or more entities are physically "associated" with one another if they interact, directly or indirectly, so that they are and/or remain in physical proximity with one another. In some embodiments, two or more entities that are physically associated with one another are covalently linked to one another; in some embodiments, two or more entities that are physically associated with one another are not covalently linked to one another but are non-covalently associated, for example by means of hydrogen bonds, van der Waals interaction, hydrophobic interactions, magnetism, and combinations thereof.

***Biological Sample:*** As used herein, the term "biological sample" typically refers to a sample obtained or derived from a biological source (e.g., a tissue or organism or cell culture) of interest, as described herein. In some embodiments, a source of interest is or comprises an organism, such as an animal or human. In some embodiments, a biological sample is or comprises biological tissue or fluid. In some embodiments, a biological sample may be or comprise bone marrow; blood; blood cells; ascites; tissue or fine needle biopsy samples; cell-containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; feces, other body fluids, secretions, and/or excretions; and/or cells therefrom, *etc.* In some embodiments, a biological sample is or comprises cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by methods selected from the group consisting of biopsy (*e.g.*, fine needle aspiration or tissue biopsy), surgery, collection of body fluid (*e.g.,* blood, lymph, feces *etc.*), *etc.* In some embodiments, as will be clear from context, the term "sample" refers to a preparation that is obtained by processing (e.g., by removing one or more components of and/or by adding one or more agents to) a primary sample. For example, filtering using a semi-permeable membrane. Such a "processed sample" may comprise, for example nucleic acids or proteins extracted from a sample or obtained by subjecting a primary sample to techniques such as amplification or reverse transcription of mRNA, isolation and/or purification of certain components, *etc.*

***Characteristic portion:*** As used herein, the term "characteristic portion", in the broadest sense, refers to a portion of a substance whose presence (or absence) correlates with presence (or absence) of a particular feature, attribute, or activity of the substance. In some embodiments, a characteristic portion of a substance is a portion that is found in the substance and in related substances that share the particular feature, attribute or activity, but not in those that do not share the particular feature, attribute or activity. In certain embodiments, a characteristic portion shares at least one functional characteristic with the intact substance. For example, in some embodiments, a "characteristic portion" of a protein or polypeptide is one that contains a continuous stretch of amino acids, or a collection of continuous stretches of amino acids, that together are characteristic of a protein or polypeptide. In some embodiments, each such continuous stretch generally contains at least 2, 5, 10, 15, 20, 50, or more amino acids. In general, a characteristic portion of a substance (*e.g.,* of a protein, antibody, *etc.*) is one that, in addition to the sequence and/or structural identity specified above, shares at least one functional characteristic with the relevant intact substance. In some embodiments, a characteristic portion may be biologically active.

***Characteristic sequence:*** A "characteristic sequence" is a sequence that is found in all members of a family of polypeptides or nucleic acids, and therefore can be used by those of ordinary skill in the art to define members of the family.

***Characteristic sequence element:*** As used herein, the phrase "characteristic sequence element" refers to a sequence element found in a polymer (e.g., in a polypeptide or nucleic acid) that represents a characteristic portion of that polymer. In some embodiments, presence of a characteristic sequence element correlates with presence or level of a particular activity or property of the polymer. In some embodiments, presence (or absence) of a characteristic sequence element defines a particular polymer as a member (or not a member) of a particular family or group of such polymers. A characteristic sequence element typically comprises at least two monomers (e.g., amino acids or nucleotides). In some embodiments, a characteristic sequence element includes at least 2, 3, 4, 5, 6, 7, 8, 9, 10, 11, 12, 13, 14, 15, 20, 25, 30, 35, 40, 45, 50, or more monomers (e.g., contiguously linked monomers). In some embodiments, a characteristic sequence element includes at least first and second stretches of contiguous monomers spaced apart by one or more spacer regions whose length may or may not vary across polymers that share the sequence element.

***Comparable:*** As used herein, the term "comparable" refers to two or more agents, entities, situations, sets of conditions, etc., that may not be identical to one another but that are sufficiently similar to permit comparison there between so that one skilled in the art will appreciate that conclusions may reasonably be drawn based on differences or similarities observed. In some embodiments, comparable sets of conditions, circumstances, individuals, or populations are characterized by a plurality of substantially identical features and one or a small number of varied features. Those of ordinary skill in the art will understand, in context, what degree of identity is required in any given circumstance for two or more such agents, entities, situations, sets of conditions, etc to be considered comparable. For example, those of ordinary skill in the art will appreciate that sets of circumstances, individuals, or populations are comparable to one another when characterized by a sufficient number and type of substantially identical features to warrant a reasonable conclusion that differences in results obtained or phenomena observed under or with different sets of circumstances, individuals, or populations are caused by or indicative of the variation in those features that are varied.

***Corresponding to:*** As used herein, the term "corresponding to" may be used to designate the position/identity of a structural element in a compound or composition through comparison with an appropriate reference compound or composition. For example, in some embodiments, a monomeric residue in a polymer (e.g., an amino acid residue in a polypeptide or a nucleic acid residue in a polynucleotide) may be identified as "corresponding to" a residue in an appropriate reference polymer.. For example, those of ordinary skill will appreciate that, for purposes of simplicity, residues in a polypeptide are often designated using a canonical numbering system based on a reference related polypeptide, so that an amino acid *"corresponding to"* a residue at position 190, for example, need not actually be the 190^{th} amino acid in a particular amino acid chain but rather corresponds to the residue found at 190 in the reference polypeptide; those of ordinary skill in the art readily appreciate how to identify *"corresponding"* amino acids. For example, those skilled in the art will be aware of various sequence alignment strategies, including software programs such as, for example, BLAST, CS-BLAST, CUSASW++, DIAMOND, FASTA, GGSEARCH/GLSEARCH, Genoogle, HMMER, HHpred/HHsearch, IDF, Infernal, KLAST, USEARCH, parasail, PSI-BLAST, PSI-Search, ScalaBLAST, Sequilab, SAM, SSEARCH, SWAPHI, SWAPHI-LS, SWIMM, or SWIPE that can be utilized, for example, to identify "corresponding" residues in polypeptides and/or nucleic acids in accordance with the present disclosure.

***Detectable entity:*** The term "detectable entity" as used herein refers to any element, molecule, functional group, compound, fragment or moiety that is detectable. In some embodiments, a detectable entity is provided or utilized alone. In some embodiments, a detectable entity is provided and/or utilized in association with (e.g., joined to) another agent. Examples of detectable entities include, but are not limited to: various ligands, radionuclides (e.g., ³H, ¹⁴C, ¹⁸F, ¹⁹F, ³²P, ³⁵S, ¹³⁵I, ¹²⁵I, ¹²³I, ⁶⁴Cu, ¹⁸⁷Re, ¹¹¹In, ⁹⁰Y, ^{99m}Tc, ¹⁷⁷Lu, ⁸⁹Zr etc.), fluorescent dyes (for specific exemplary fluorescent dyes, see below), chemiluminescent agents (such as, for example, acridinum esters, stabilized dioxetanes, and the like), bioluminescent agents, spectrally resolvable inorganic fluorescent semiconductors nanocrystals (i.e., quantum dots), metal nanoparticles (e.g., gold, silver, copper, platinum, etc.) nanoclusters, paramagnetic metal ions, enzymes (for specific examples of enzymes, see below), colorimetric labels (such as, for example, dyes, colloidal gold, and the like), biotin, dioxigenin, haptens, and proteins for which antisera or monoclonal antibodies are available.

***Engineered:*** In general, the term "engineered" refers to the aspect of having been manipulated by the hand of man. For example, a polynucleotide is considered to be "engineered" when two or more sequences, that are not linked together in that order in nature, are manipulated by the hand of man to be directly linked to one another in the engineered polynucleotide. For example, in some embodiments of the present invention, an engineered polynucleotide comprises a regulatory sequence that is found in nature in operative association with a first coding sequence but not in operative association with a second coding sequence, is linked by the hand of man so that it is operatively associated with the second coding sequence. Comparably, a cell or organism is considered to be "engineered" if it has been manipulated so that its genetic information is altered (*e.g.*, new genetic material not previously present has been introduced, for example by transformation, mating, somatic hybridization, transfection, transduction, or other mechanism, or previously present genetic material is altered or removed, for example by substitution or deletion mutation, or by mating protocols). As is common practice and is understood by those in the art, progeny of an engineered polynucleotide or cell are typically still referred to as "engineered" even though the actual manipulation was performed on a prior entity.

***Marker:*** A marker, as used herein, refers to an entity or moiety whose presence or level is a characteristic of a particular state or event. In some embodiments, presence or level of a particular marker may be characteristic of presence or stage of a disease, disorder, or condition. To give but one example, in some embodiments, the term refers to a gene expression product that is characteristic of a particular tumor, tumor subclass, stage of tumor, etc. Alternatively or additionally, in some embodiments, a presence or level of a particular marker correlates with activity (or activity level) of a particular signaling pathway, for example that may be characteristic of a particular class of tumors. The statistical significance of the presence or absence of a marker may vary depending upon the particular marker. In some embodiments, detection of a marker is highly specific in that it reflects a high probability that the tumor is of a particular subclass. Such specificity may come at the cost of sensitivity (i.e., a negative result may occur even if the tumor is a tumor that would be expected to express the marker). Conversely, markers with a high degree of sensitivity may be less specific that those with lower sensitivity. According to the present invention a useful marker need not distinguish tumors of a particular subclass with 100% accuracy.

***Nucleic acid*:** As used herein, in its broadest sense, refers to any compound and/or substance that is or can be incorporated into an oligonucleotide chain. In some embodiments, a nucleic acid is a compound and/or substance that is or can be incorporated into an oligonucleotide chain via a phosphodiester linkage. As will be clear from context, in some embodiments, *"nucleic acid"* refers to an individual nucleic acid residue (e.g., a nucleotide and/or nucleoside); in some embodiments, *"nucleic acid"* refers to an oligonucleotide chain comprising individual nucleic acid residues. In some embodiments, a *"nucleic acid"* is or comprises RNA; in some embodiments, a *"nucleic acid"* is or comprises DNA. In some embodiments, a nucleic acid is, comprises, or consists of one or more natural nucleic acid residues. In some embodiments, a nucleic acid is, comprises, or consists of one or more nucleic acid analogs. In some embodiments, a nucleic acid analog differs from a nucleic acid in that it does not utilize a phosphodiester backbone. For example, in some embodiments, a nucleic acid is, comprises, or consists of one or more *"peptide nucleic acids",* which are known in the art and have peptide bonds instead of phosphodiester bonds in the backbone, are considered within the scope of the present invention. Alternatively or additionally, in some embodiments, a nucleic acid has one or more phosphorothioate and/or 5'-N-phosphoramidite linkages rather than phosphodiester bonds. In some embodiments, a nucleic acid is, comprises, or consists of one or more natural nucleosides (e.g., adenosine, thymidine, guanosine, cytidine, uridine, deoxyadenosine, deoxythymidine, deoxy guanosine, and deoxycytidine). In some embodiments, a nucleic acid is, comprises, or consists of one or more nucleoside analogs (e.g., 2-aminoadenosine, 2-thiothymidine, inosine, pyrrolo-pyrimidine, 3 -methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 0(6)-methylguanine, 2-thiocytidine, methylated bases, intercalated bases, and combinations thereof). In some embodiments, a nucleic acid comprises one or more modified sugars (e.g., 2'-fluororibose, ribose, 2'-deoxyribose, arabinose, and hexose) as compared with those in natural nucleic acids. In some embodiments, a nucleic acid has a nucleotide sequence that encodes a functional gene product such as an RNA or protein. In some embodiments, a nucleic acid includes one or more introns. In some embodiments, nucleic acids are prepared by one or more of isolation from a natural source, enzymatic synthesis by polymerization based on a complementary template (*in vivo* or *in vitro*), reproduction in a recombinant cell or system, and chemical synthesis. In some embodiments, a nucleic acid is at least 3, 4, 5, 6, 7, 8, 9, 10, 15, 20, 25, 30, 35, 40, 45, 50, 55, 60, 65, 70, 75, 80, 85, 90, 95, 100, 1 10, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000 or more residues long. In some embodiments, a nucleic acid is partly or wholly single stranded; in some embodiments, a nucleic acid is partly or wholly double stranded. In some embodiments a nucleic acid has a nucleotide sequence comprising at least one element that encodes, or is the complement of a sequence that encodes, a polypeptide. In some embodiments, a nucleic acid has enzymatic activity. Those skilled in the art, reading the present specification, will appreciate that ligation oligonucleotide sets, activating nucleic acids, and/or guide RNAs can each be engineered and/or manipulated, e.g., to incorporate nucleotide analogs, etc.

***Operably linked*:** as used herein, refers to a juxtaposition wherein the components described are in a relationship permitting them to function in their intended manner. A control element "*operably linked*" to a functional element is associated in such a way that expression and/or activity of the functional element is achieved under conditions compatible with the control element. In some embodiments, *"operably linked"* control elements are contiguous (e.g., covalently linked) with the coding elements of interest; in some embodiments, control elements act in *trans* to or otherwise at a from the functional element of interest.

***Polypeptide*:** As used herein refers to any polymeric chain of amino acids. In some embodiments, a polypeptide has an amino acid sequence that occurs in nature. In some embodiments, a polypeptide has an amino acid sequence that does not occur in nature. In some embodiments, a polypeptide has an amino acid sequence that is engineered in that it is designed and/or produced through action of the hand of man. In some embodiments, a polypeptide may comprise or consist of natural amino acids, non-natural amino acids, or both. In some embodiments, a polypeptide may comprise or consist of only natural amino acids or only non-natural amino acids. In some embodiments, a polypeptide may comprise D-amino acids, L-amino acids, or both. In some embodiments, a polypeptide may comprise only D-amino acids. In some embodiments, a polypeptide may comprise only L-amino acids. In some embodiments, a polypeptide may include one or more pendant groups or other modifications, e.g., modifying or attached to one or more amino acid side chains, at the polypeptide's N-terminus, at the polypeptide's C-terminus, or any combination thereof. In some embodiments, such pendant groups or modifications may be selected from the group consisting of acetylation, amidation, lipidation, methylation, pegylation, etc., including combinations thereof. In some embodiments, a polypeptide may be cyclic, and/or may comprise a cyclic portion. In some embodiments, a polypeptide is not cyclic and/or does not comprise any cyclic portion. In some embodiments, a polypeptide is linear. In some embodiments, a polypeptide may be or comprise a stapled polypeptide. In some embodiments, the term "polypeptide" may be appended to a name of a reference polypeptide, activity, or structure; in such instances it is used herein to refer to polypeptides that share the relevant activity or structure and thus can be considered to be members of the same class or family of polypeptides. For each such class, the present specification provides and/or those skilled in the art will be aware of exemplary polypeptides within the class whose amino acid sequences and/or functions are known; in some embodiments, such exemplary polypeptides are reference polypeptides for the polypeptide class or family. In some embodiments, a member of a polypeptide class or family shows significant sequence homology or identity with, shares a common sequence motif (e.g., a characteristic sequence element) with, and/or shares a common activity (in some embodiments at a comparable level or within a designated range) with a reference polypeptide of the class; in some embodiments with all polypeptides within the class). For example, in some embodiments, a member polypeptide shows an overall degree of sequence homology or identity with a reference polypeptide that is at least about 30-40%, and is often greater than about 50%, 60%, 70%, 80%, 90%, 91%, 92%, 93%, 94%, 95%, 96%, 97%, 98%, 99% or more and/or includes at least one region (e.g., a conserved region that may in some embodiments be or comprise a characteristic sequence element) that shows very high sequence identity, often greater than 90% or even 95%, 96%, 97%, 98%, or 99%. Such a conserved region usually encompasses at least 3-4 and often up to 20 or more amino acids; in some embodiments, a conserved region encompasses at least one stretch of at least 2, 3, 4, 5, 6, 7, 8, 9, 10, 11, 12, 13, 14, 15 or more contiguous amino acids. In some embodiments, a relevant polypeptide may comprise or consist of a fragment of a parent polypeptide. In some embodiments, a useful polypeptide as may comprise or consist of a plurality of fragments, each of which is found in the same parent polypeptide in a different spatial arrangement relative to one another than is found in the polypeptide of interest (e.g., fragments that are directly linked in the parent may be spatially separated in the polypeptide of interest or vice versa, and/or fragments may be present in a different order in the polypeptide of interest than in the parent), so that the polypeptide of interest is a derivative of its parent polypeptide.

***Reference:*** As used herein describes a standard or control relative to which a comparison is performed. For example, in some embodiments, an agent, animal, individual, population, sample, sequence or value of interest is compared with a reference or control agent, animal, individual, population, sample, sequence or value. In some embodiments, a reference or control is tested and/or determined substantially simultaneously with the testing or determination of interest. In some embodiments, a reference or control is a historical reference or control, optionally embodied in a tangible medium. Typically, as would be understood by those skilled in the art, a reference or control is determined or characterized under comparable conditions or circumstances to those under assessment. Those skilled in the art will appreciate when sufficient similarities are present to justify reliance on and/or comparison to a particular possible reference or control.

***Specific binding:*** As used herein, the term "specific binding" refers to an ability to discriminate between possible binding partners in the environment in which binding is to occur. A binding agent that interacts with one particular target when other potential targets are present is said to *"bind specifically"* to the target with which it interacts. In some embodiments, specific binding is assessed by detecting or determining degree of association between the binding agent and its partner; in some embodiments, specific binding is assessed by detecting or determining degree of dissociation of a binding agent-partner complex; in some embodiments, specific binding is assessed by detecting or determining ability of the binding agent to compete an alternative interaction between its partner and another entity. In some embodiments, specific binding is assessed by performing such detections or determinations across a range of concentrations.

### Detailed Description of Certain Embodiments

Recently, various nucleic acid detection and/or manipulation systems have described which utilize the so-called "collateral cleavage" activity present in certain Cas enzymes. These systems include, for example, the SHERLOCK^{™} systems that utilize Cas13 (and/or Csm6 enzymes) and are described, for example, in: WO2018/107129 to Abudayeh et al; Gootenberg et al. Science 356:438, 2017; Gootenberg et al, Science 360:439, 2018, and the DETECTR^{™} systems that utilize Cas12 enzymes and are described, for example, in WO2017/218573 to Doudna; Chen et al. Science 360:436, 2018. Reviews of these systems in prominent journals describe them a "major advance" (Kocak & Gersbach Nature 557:168, 2018) and as "revolutionizing the field of molecular diagnostics" (Chertow Science 360:381, 2018). Furthermore, it is becoming increasingly clear that collateral activity is a feature of many Cas enzymes, and technologies are available for readily identifying those displaying such activity (see, for example, Harrington et al., Science 362:839, 2018, which describes Cas14 loci and enzymes therein).

Broadly speaking, existing systems that utilize Cas collateral cleavage activity to detect a nucleic acid require a Cas enzyme whose gRNA has been engineered to hybridize with a particular target sequence of interest. When such a Cas enzyme (and its gRNA) is contacted with a sample containing a nucleic acid with the target site of interest, the Cas enzyme's collateral activity is activated; such activation can be detected by including a probe labeled in such a way that its cleavage is detectable. To give but one example, when the probe is an oligonucleotide labeled with both a fluorophore and a quencher, cleavage of the oligonucleotide releases the fluorophore from the quencher and generates a detectable signal. The SHERLOCK^{™} and DETECTR^{™} systems noted herein have been described as being able to achieve attomolar sensitivity when combined with target amplification, and in particular with isothermal amplification (see, Gootenberg et al. Science 360:439, 2018 and Chen et al. Science 360:436, 2018).

The present disclosure appreciates that the described SHERLOCK^{™} and DETECTR^{™} systems have many powerful attributes. The present disclosure, however, also identifies the source of certain problem(s) with these systems, including that they require design of a new guide RNA for each target nucleic acid of interest; the present disclosure notes that this requirement for target-specific gRNAs can impose significant burdens on development, as individual gRNA-target pairs may need to be optimized for use together and/or with particular Cas enzymes.

The present disclosure further notes that SHERLOCK^{™} and DETECTR^{™} systems are reported to achieve extreme sensitivity only when used together with amplification of original target nucleic acid. The present disclosure teaches that a requirement for initial target amplification could be the source of a problem, as it might introduce mutations that could generate false positive and/or false negative results.

The present disclosure solves these problems, and furthermore discovers various unexpected and surprising advantages of de-coupling target sequence from activation of Cas cleavage.

In various embodiments, the present disclosure provides systems that utilize ligation-based technologies for initial (and target-sequence dependent) hybridization to target nucleic acids of interest, and uses those systems to generate a Cas-activating nucleic acid that triggers Cas cleavage (e.g., collateral cleavage) activity. Because the Cas-activating nucleic acid is only generated if and when the target nucleic acid of interest is present, the Cas guide RNA need not be specific for the target sequence, but rather can bind elsewhere in the activating nucleic acid.

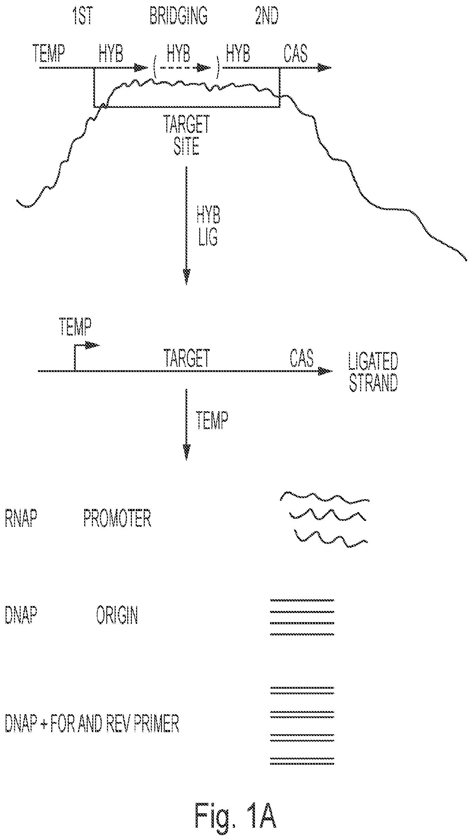

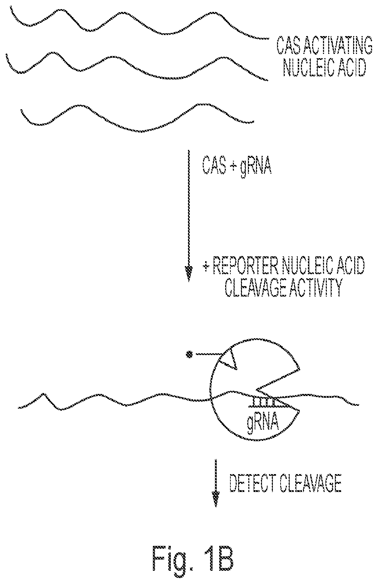

*Figures 1A* and *1B**,* together, depict an exemplary embodiment of nucleic acid detection in accordance with the present disclosure. As indicated in *Figure 1A**,* a nucleic acid sample that may include at least one nucleic acid of interest (i.e., whose nucleotide sequence is or includes a target site of interest) is contacted with a set of ligation oligonucleotides, which set comprises (i) a first ligation oligonucleotide whose nucleotide sequence includes templating element and a first target hybridization element; (ii) a second ligation oligonucleotide whose nucleotide sequence includes a second target hybridization element and a Cas recognition element; and (iii) optionally one or more bridging oligonucleotides whose nucleotide sequence is or comprises one or more additional target hybridization elements. The oligonucleotides of the set are selected so that, when they are simultaneously hybridized to the nucleic acid of interest, they abut one another so that a ligase enzyme can link them to form a single ligated strand. The ligated strand is or is a complement or component (e.g., is one strand of a double-stranded entity) of a nucleic acid that activates cleavage (e.g., collateral cleavage) by at least one Cas protein.

In principle, the ligated strand itself could be utilized as a Cas-activating nucleic acid (in which case the templating element would not be required). However, in many embodiments, as depicted in *Figure 1A**,* the ligated strand is copied by a system that acts on the templating element. For example, where the templating element is or comprises a promoter (or its complement) and/or one or more transcriptional regulatory elements (or complements thereof), the system may be or comprise an RNA polymerase; where the templating element is or comprises an origin of replication and/or a binding site (or complement thereof) for an extendible primer, the system may be or comprise a DNA polymerase (which, in some embodiments, may be a thermostable DNA polymerase, particularly if the ligated strand includes a sequence element corresponding to a second extendible primer and the system includes an appropriate pair of primers to amplify a duplex of the ligated strand and its complement). Such copying generates a population of nucleic acid molecules referred to in this Example and in *Figures 1A* and *1B* as "Cas activating nucleic acids" because they include a sequence complementary to a crRNA/gRNA for a Cas protein with cleavage (e.g., collateral cleavage) activity that will be detected. As depicted in *Figure 1B**,* when an appropriate Cas for the type of nucleic acid (i.e., RNA, ssDNA, or dsDNA) present in the Cas activating nucleic acid is contacted with the Cas target nucleic acid, its cleavage (e.g., collateral cleavage) activity is activated, and an appropriate reporter nucleic acid is cleaved, resulting in a detectable signal.

Below, certain features and/or attributes of various elements and/or embodiments of the present invention are discussed in more detail. Those of ordinary skill will also appreciate that, given the templating property of nucleic acid strands, and their ability to hybridize with one another, it is conventional in the field to refer to a particular "sequence element" or "site" and relying on the skilled artisan to understand from context when the "forward" or "sense" form is being referenced, and when its complement (the "reverse" or "antisense" form).

### Target Nucleic Acid

Those skilled in the art will immediately appreciate that technologies provided herein are broadly applicable to achieve detection of a wide range of nucleic acids including, for example, nucleic acids from an infectious agent (e.g., a virus, microbe, parasite, etc), nucleic acids indicative of a particular physiological state or condition (e.g., presence or state of a disease, disorder or condition such as, for example, cancer or an inflammatory or metabolic disease, disorder or condition, etc), prenatal nucleic acids, etc.

In many embodiments, provided technologies are particularly useful or applicable for detection of low-abundance (e.g., less than about 10 fM, or about 1 fM, or about 100 aM) nucleic acids.

Typically, provided technologies will be applied to one or more samples to assess presence and/or level of one or more target nucleic acids in the sample. In some embodiments, the sample is a biological sample; in some embodiments, a sample is an environmental sample.

In some embodiments, a sample will be processed (e.g., nucleic acids will be partially or substantially isolated or purified out of a primary sample); in some embodiments, only minimal processing will have been performed (i.e., the sample will be a crude sample).

### Ligation Oligonucleotide Sets

As described herein, the present disclosure utilizes ligation technologies for hybridization with/detection of initial target nucleic acids. The ligation step is therefore sequence-specific to the target sequence of interest; for each such target site, a set of ligation oligonucleotides is designed that together hybridize across a target sequence of interest, adjacent to one another so that activity of a ligase links hybridized oligonucleotides together to form a single ligated strand. This ligated strand includes the complement of the entire target site selected, and also includes a Cas recognition element, and typically a templating element, that, prior to ligation, were not part of the same oligonucleotide.

Those skilled in the art will be aware of a variety of systems that utilize sets of ligation oligonucleotides to bring together functional elements located on separate oligonucleotides of the set only when the set becomes linked by ligation, as occurs in the presence of a target nucleic acid of interest, to which each of the oligonucleotides in the set hybridizes at adjacent portions of a target site, is present. (see, for example, promoter-ligation-activated-transcription amplification of nucleic acid sequences, as described in US Patent Number 5194370; multiplex ligatable probe amplification [MLPA], as described in US Patent 6955901, and Nucleic Acids Research 30:e27, 2002; RNA-splinted polynucleotide ligase activity as described, for example, in Nucleic Acids Research 42:1831, 2013, US patent application US2014/0179539 and Nucleic Acids Research 33:e116, 2016). Those of ordinary skill are therefore well aware of design parameters relevant to construction of oligonucleotides within oligonucleotide sets as described herein (see also US patent application US2008/0090238).

### Target Hybridization Elements

As depicted in *Figure 1**,* ligation oligonucleotide sets for use in accordance with the present disclosure are designed so that each includes a sequence element that hybridizes to a target nucleic acid at a position adjacent to that where another oligonucleotide of the set, such that when all oligonucleotides of a particular set are hybridized to a target nucleic acid (i.e., to a nucleic acid that includes a target site), they can be covalently linked to one another by a ligase.

In some embodiments, a set of ligation oligonucleotides includes only two (i.e., first and second) oligonucleotides, each of which includes a target hybridization element and one of which includes a Cas recognition element; in some embodiments, the other includes a templating element. In some embodiments, a set of ligation oligonucleotides includes one or more bridging oligonucleotides that hybridize to the target site between the first and second oligonucleotides.

### Target Recognition Elements

Those skilled in the art, reading the present disclosure, will appreciate that the target site can be any sequence of interest - e.g., whose presence in a particular sample is to be assessed. Those skilled in the art will further be aware (e.g., from other ligation systems described in the literature, including those cited herein) of design considerations relevant to selecting length and/or sequence characteristics (e.g., GC content etc) of individual target hybridization elements in oligonucleotides within a set of ligation oligonucleotides.

### Cas Recognition Elements

Those skilled in the art, reading the present disclosure, will further appreciate that a Cas recognition element is a sequence (or, in some embodiments, the complement thereof) that is bound by a Cas gRNA and can be designed, selected, and/or optimized particularly for use in detection systems as described herein. As already noted, one feature of provided technologies is that it is not necessary to design or utilize a different Cas recognition element for each target nucleic acid; the Cas recognition element typically does not hybridize to the target nucleic acid during the ligation step.

In some embodiments of the present disclosure, for example, where multiple ligation oligonucleotide sets (i.e., designed for detection of different target nucleic acids) are to be used simultaneously (e.g., together in the same reactions), it may be desirable that different ligation oligonucleotide sets have different Cas recognition elements (e.g., each ligation oligonucleotide set may have its own Cas recognition element), but in some embodiments, multiple different ligation oligonucleotide sets, or even all ligation oligonucleotide sets, may include the same Cas recognition element.

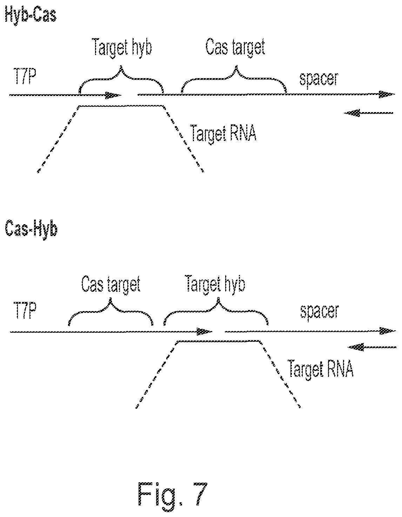

Those skilled in the art, reading the present disclosure, will appreciate that, in some embodiments, a Cas recognition element can precede, come before, be upstream of, and/or be 5' of, a target recognition element. In some embodiments, a Cas recognition element can follow, succeed, come after, be downstream of, and/or be 3' of, a target recognition element. See, for example, Figure 7.

### Templating Elements

At least one oligonucleotide within a ligation oligonucleotide set will typically include a templating element, which permits and/or directs templating of the ligated strand.

In many embodiments, a templating element will be or comprise a binding site for an extendable oligonucleotide (i.e., a primer). Incubation of the ligated strand with such an oligonucleotide and an appropriate extending enzyme (e.g., a DNA polymerase) generates a template strand, so that a duplex nucleic acid is created.

In some embodiments, such a duplex nucleic acid includes a binding site second extendable oligonucleotide (i.e., a second primer), so that the duplex can be amplified by polymerase chain reaction; in such embodiments, the binding site for the second extendable oligonucleotide is typically in the second oligonucleotide, on the far end of the Cas recognition element, relative to the first oligonucleotide, so that when the duplex is incubated with the first and second primer (or a different primer pair, so long as it spans the Cas recognition element) and a DNA polymerase (e.g., a thermostable DNA polymerase), multiple copies of a duplex comprising the Cas recognition element are generated.

A templating element may be or comprise a promoter or complement thereof; may be or comprise one or more transcriptional regulatory elements (e.g., enhancers, repressor binding sites, etc), so that multiple RNA templates (or copies) of the ligated strand are generated by incubating, e.g., a duplex as described above, with an RNA polymerase; may be or comprise a binding site for a first extendible primer.

A templating element may be or comprise an origin of replication, so that multiple DNA templates (or copies) of the ligated strand are generated by incubation with a DNA polymerase.

Those of ordinary skill in the art, reading the present disclosure, will appreciate that an appropriate templating element for use in a particular embodiment may be selected to generate (or permit ready generation of - e.g., by denaturation) a templated product (e.g., RNA, ssDNA, or dsDNA) that is effective as a Cas activating nucleic acid for whichever Cas enzyme (e.g., Cas9, Cas12, Cas13, Cas14, etc) will be utilized in the cleavage processes described herein.

### Activating Nucleic Acid

An "activating" nucleic acid, as that term is used in the present disclosure, is a nucleic acid to which a gRNA hybridizes, thereby activating the relevant Cas enzyme (and, more specifically, its collateral activity).

Those skilled in the art will be aware that different Cas enzymes are activated by different types of activating nucleic acids (e.g., RNA, ssDNA, dsDNA, or combinations thereof), and will readily be able to design systems in accordance with the present disclosure that generate an activating nucleic acid appropriate to a particular utilized Cas enzyme.

Those skilled in the art will be aware, for example, that Cas 13 enzymes are often activated by RNA, whereas Cas12 enzymes are often activated by DNA.

In some embodiments of the present disclosure, a Cas activating nucleic acid is or comprises RNA. In some embodiments, a Cas activating nucleic acid is or comprises ssDNA. In some embodiments, a Cas activating nucleic acid is or comprises dsDNA.

In some embodiments, a Cas activating nucleic acid is not an amplified target nucleic acid.

Typically, an activating nucleic acid in accordance with the present disclosure, is generated via action of the templating element(s).

### Cas Enzymes

Cas enzymes were originally identified as part of the CRISPR (which stands for "clusters of regularly interspaced short palindromic repeats")-Cas (which stands for "CRISPR-associated") systems that provide microbes with adaptive immunity to infectious nucleic acids. Those skilled in the art are aware of an enormous number of Cas enzymes, and of sequence elements and functional characteristics that categorize them into different classes. Class 1 CRISPR-Cas systems have multi subunit effector complexes; Class 2 systems have single-subunit effectors.

At least six different "Types" of Cas proteins have been described; Types I, II, and IV are Class 1 enzymes whereas Types II (including Cas9), V (including Cas12 and Cas14), and VI (including Cas 13) are Class 2 enzymes. Technologies for identifying Cas enzymes, and classifying them (e.g., based on presence, organization, and/or sequence of a RuvC domain and/or one or more other sequence elements) are by now well known in the art. Moreover, many Cas variants have been prepared, and those skilled in the art have a good understanding of structural (e.g., sequence) elements that participate in (e.g., are necessary and/or sufficient for) activities of Cas enzymes. Those skilled in the art, reading the present application, will appreciate that provided technologies can utilize, in various embodiments, any Cas enzyme (or variant, e.g., engineered variant, thereof) that has cleavage activity which is appropriate to the read-out to be utilized and which is activated by gRNA binding. Moreover, those skilled in the art, reading the present disclosure, will be well familiar with design choices etc appropriate to match, for example, a particular type of Cas with a particular Cas-activating nucleic acid and/or cleavage substrate (e.g., probe).

### Collateral Cleavage

Certain Cas enzymes, specifically including Certain Type V and Type VI Cas enzymes, such as Cas12, Cas13, and Cas14 (e.g., Cpf1/Cas12a, C2c2/Cas13a, Cas 13b, Cas13c, Cas14a, etc) have been demonstrated to have non-specific nuclease activity that is activated when their gRNA/crRNA binds to its target. This non-specific cleavage activity is often referred to as "collateral cleavage".

As noted above, nucleic acid detection systems have recently been developed that utilize the collateral cleavage activity of a Cas protein to detect presence of a target nucleic acid (or, more accurately, a nucleic acid whose nucleotide sequence includes a target site) of interest. In many embodiments, the present utilizes a Cas protein with collateral activity, and detects activation of that activity/cleavage of a probe.

### Site-specific Cleavage

In certain embodiments, the present disclosure may utilize a Cas enzyme that cleaves a substrate other than by collateral cleavage; those skilled in the art will appreciate that many or most Cas enzymes display site-specific cleavage activity, often at or near the its gRNA binding site.

By providing technologies that de-couple Cas activation from target nucleic acid sequence hybridization, the present disclosure permits use of a site-specific Cas cleavage readout system while still avoiding the need to engineer a new gRNA for each nucleic acid target of interest. Thus, in some embodiments, site-specific Cas cleavage may be used as a read-out; in some such embodiments, the relevant Cas (e.g., Cas9) does not have collateral cleavage activity.

Those skilled in the art are aware of a variety of systems that have been developed and/or can be used to detect Cas site-specific cleavage activity. To give but a few examples, cleavage of an inactivating DNA sequence from a dsDNA expression cassette enabling cell-free reporter expression, or other means of detecting a dsDNA break may be employed.

### Guide RNA

Cas enzymes are activated to cleave (whether specifically or non-specifically) nucleic acids when their guide RNAs hybridize with a complementary sequence (herein referred to as "Cas recognition element"). It is well established that guide RNAs can be engineered by researchers to hybridize with any sequence of interest. Additionally, it is well established that guide RNAs may include natural nucleotides, nucleotide analogs, and/or combinations thereof. All of that established knowledge is relevant to, and may be employed in the practice of, the present disclosure. A guide RNA capable of binding to said Cas recognition element.

For example, those skilled in the art will appreciate that a guide RNA may, in some embodiments, have a length (and/or a portion that hybridizes to a Cas recognition element) that is within a range of about 16-28 nucleotides (e.g., about 16, about 17, about 18, about 19, about 20, about 21, about 22, about 23, about 24, about 25, about 26, about 27, or about 28 nucleotides).

One skilled in the art will also appreciate that, in certain embodiments, a guide RNA may have less than 100% perfect complementarity with a relevant Cas recognition element (e.g., may be about 90%, about 91%, about 92%, about 93%, about 94%, about 95%, about 96%, about 97%, about 98%, about 99%, or 100% complementary).

One feature of systems described herein is that, in certain embodiments, they may be designed and/or utilized to detect target nucleic acids that do not include a sequence element complementary to the guide RNA of a Cas enzyme with collateral cleavage activity. Indeed, one advantage of provided technologies is that they do not require that a different guide RNA be engineered for each target nucleic acid of interest. Rather, one or more effective (e.g., optimized and/or otherwise desirable) guide RNA/Cas recognition element pairs may be provided and/or utilized to detect a variety of different target nucleic acids of interest.

### Probe

In accordance with the present disclosure, cleavage (e.g., collateral cleavage) activity of a Cas enzyme may be detected by detecting cleavage of an appropriate probe. Those skilled in the art are aware that different Cas enzymes may demonstrate cleavage (and particularly collateral cleavage) activity with respect to different probes - for instance, Cas13 enzymes typically collaterally cleave RNA probes; Cast2 enzymes typically cleave ssDNA probes. Cas9 enzymes usually do not have collateral activity and cleave dsDNA substrates. Those skilled in the art, reading the present disclosure, will readily be able to design, select, and/or produce appropriate probes susceptible to collateral cleavage by particular Cas enzymes.

Typically, a probe for use in accordance with the present disclosure is characterized in that its cleavage can be detected. Those skilled in the art are aware of a variety of strategies for and embodiments of probes whose collateral cleavage by a particular Cas enzyme is detectable. To give but one example, in some embodiments, a probe may be labeled with a fluorescence-emitting-dye pair (e.g., a FRET pair or a fluor/quencher pair), such that a change (e.g., an increase - such as when cleavage relieves quenching, a decrease, a change in wavelength, or combinations thereof) in fluorescence is observed when the probe is cleaved. Appropriate FRET pairs are known in the art (see, for example, Bajar et al sensors (Basel), 2016; Abraham et al. PLoS One 10:e0134436, 2015).

Various other strategies for detecting cleavage of a probe are also known in the art and include, for example, masking constructs as described with respect to SHERLOCK^{™} (see, e.g., WO 2018/107129).

Alternatively or additionally, in some embodiments, a probe may be an appropriate nucleic acid coupled to a surface, so that cleavage of the probe can be detected by, for example, detecting DNA- or RNA-conjugated particle proximity to a surface.

### Applications

Those skilled in the art, reading the present specification, will immediately appreciate that technology it provides is useful in a wide range of contexts, and can be applied in a variety of formats.

In some embodiments, one or more components (e.g., target nucleic acid, ligation oligonucleotide(s), ligated strand, Cas activating nucleic acid, Cas, and combination thereof) may be associated with (e.g., attached to) a solid support.

In some embodiments, a plurality of ligation oligonucleotide sets is utilized substantially simultaneously, so that multiple target nucleic acids may be detected contemporaneously.

In some embodiments, provided technologies may be multiplexed, for example, utilizing different Cas enzymes (and/or readouts) for different target nucleic acid sequences and/or different ligation oligonucleotides.

### Exemplification

### Example 1: Exemplary system for sensitive, specific detection of nucleic acid using ligation-based transcriptional activation and CRISPR collateral activity.

The present Example describes an exemplary system for sensitive, specific detection of nucleic acid using ligation-based transcriptional activation and CRISPR collateral activity. Particularly noteworthy among the attributes of this provided system are that (i) it can decouple activation of CRISPR collateral activity from the target sequence to be detected; and/or (ii) it can provide sensitive and specific detection, down to single base discrimination, even without use of amplification technologies (e.g., isothermal amplification).

As noted herein, recently-developed systems which utilize the so-called "collateral cleavage" activity that has been identified for certain Cas enzymes to detect nucleic acids have been described as a "major advance" (Kocak & Gersbach Nature 557:168, 2018) and as "revolutionizing the field of molecular diagnostics" (Chertow Science 360:381, 2018). Literature describing these systems often emphasizes the benefits of, or even need for, use of amplification technologies (in particular highlighting certain isothermal amplification technologies such as, for example, recombinase polymerase amplification, RPA), which are applied to a target sample to amplify target nucleic acid in order to achieve ultra-high sensitivity nucleic acid detection (see, for example, SHERLOCK^{™} systems described, for example, in: WO2018/107129 to Abudayeh et al; Gootenberg et al. Science 356:438, 2017; Gootenberg et al, Science 360:439, 2018; and DETECTR^{™} systems described, for example, in WO2017/218573 to Doudna; Chen et al. Science 360:436, 2018; including as reviewed, for example, in Chertow Science 360:381, 2018 and Kocak & Gersbach Nature 557:168, 2018).

The present disclosure, among other things, recognizes that reliance on such amplification might be problematic and/or undesirable in certain circumstances. Moreover, the present disclosure provides systems, such as described in this Example, that provide various benefits, and do not utilize such target-nucleic-acid amplification.

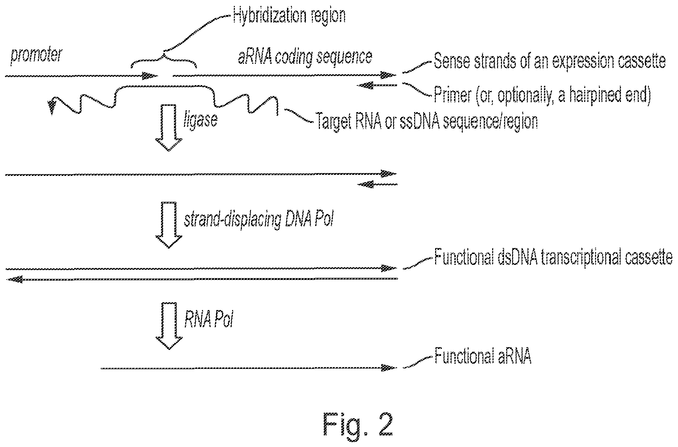

In SHERLOCK^{™} systems described in the literature, a target sequence is isothermally amplified and then detected by, e.g., Cas13a and a designed crRNA; this detection activates Cas13a collateral cleavage activity and subsequent cleavage of a reporter molecule (e.g., fluor-quencher linked by RNA). The present Example describes technology, e.g., as depicted in *Figure 2*, that can decouple collateral cleavage activity from a target sequence. In the embodiment depicted in *Figure 2**,* such decoupling is achieved by production of the crRNA-compatible sequence (which may be referred to as an "activation RNA", and is labeled "aRNA" in *Figure 2*) based on the presence of a target sequence. aRNA is produced through cell-free transcription activated by ligation of two molecules of single stranded DNA bridged by the target sequence (i.e., juxtaposed by hybridization to the target sequence). Ligation, followed by second strand synthesis, generates a functional dsDNA transcriptional cassette enabling promoter-driven transcription of the aRNA.

Advantages of this system include, for example, that (i) pre-amplification via commonly practiced isothermal amplification may be eliminated (due to aRNA amplification via cell-free transcription); (ii) design of primers specific to the target sequence is not necessary, (iii) both DNA and RNA can be used to activate expression of aRNA; (iv) ligation specificity enables single base discrimination; (v) the aRNA may have repeats of the Cas activation sequence (i.e., that bound by the crRNA), which can potentially permit activation of multiple Cas enzymes from a single aRNA; and (vi) combinations thereof.

In the particular embodiment depicted in *Figure 2*, two sense strands of DNA that, together, make up a transcriptional cassette (including a promoter and an aRNA expression sequence) are used to detect a target sequence such that the target sequence bridges the two single strands. The 3' end of the upstream strand includes sequence that hybridizes to a region of the target sequence and the 5' end of downstream strand includes sequence that hybridizes to a contiguous region of the target sequence, together with a 5' phosphate to enable ligation. When a target sequence is present, it bridges the two strands, thereby enabling ligation by a ligase enzyme (e.g., Chlorella virus DNA Ligase or T4 DNA ligase). Second strand synthesis (e.g., from an included primer, or hairpined end) via a strand-displacing DNA polymerase generates a double stranded DNA transcriptional cassette that is functional for transcription of the aRNA (e.g., via T7 transcription). This functional aRNA activates collateral activity of an appropriate Cas enzyme (e.g., Cas13a), whose crRNA binds to the aRNA coding sequence.

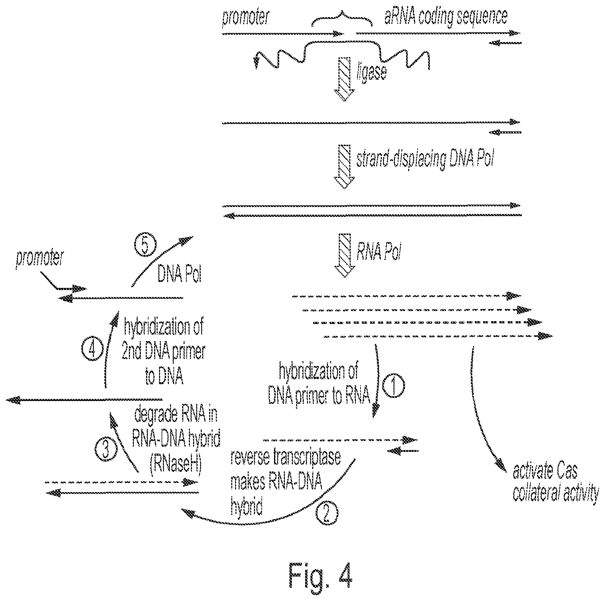

In certain embodiments, rate and/or amount of aRNA produced can be increased, for example by incorporating one or more additional components into the utilized system. For example, in some embodiments (e.g., as depicted in *Figure 4*), a reverse transcriptase and an endonuclease that catalyzes hydrolytic cleavage of RNA in an RNA-DNA hybrid may be utilized. As can be seen with reference to *Figure 4*, in the step labeled "step 4", the primer may be a separate designed primer with appropriate 5' promoter sequence, or may be the same molecule as the upstream sensor molecule depicted at the top of the figure; such post-ligation amplification leverages technologies previously described as Transcription Mediated Amplification (TMA), and Nucleic Acid Sequence-Based Amplification (NASBA).

### Example 2: An exemplary multiplexed system

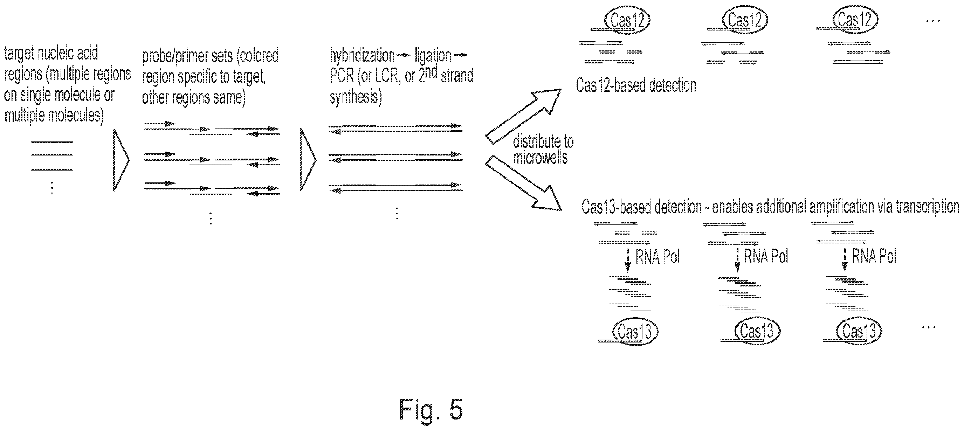

Figure 5 presents an exemplary multiplexed assay as described herein - i.e., one in which multiple target nucleic acids may be detected, e.g., simultaneously. As shown, multiple target nucleic acids, which may be on multiple different nucleic acid molecules or may represent multiple regions (e.g., different genes or sites, or different regions within a single gene) on a single nucleic acid molecule are targeted by different probe/primer sets.

Multiple ligation oligonucleotide sets, each directed to a particular target nucleic acid, are contacted with a sample and are ligated. Optionally, one or more second strand(s), each complementary with a particular ligation product, is/are synthesized. In some embodiments, a plurality of such second strands are synthesized. In some such embodiments, two or more such second strands, and optionally all second strands, are generated by extension of the same primer, which hybridizes to a sequence present in one primer of each of the relevant sets of ligation primers. Still further optionally, in some embodiments, additional copies of one or both strands of the duplex formed by such second strand synthesis is generated, for example by amplification such as PCR or LCR. Again, in some embodiments, common primer(s) may be used for synthesis of copies of two or more, or optionally all, ligated strands.

In some embodiments, reagents for ligation and (optional) second strand synthesis and/or amplification are included in a "single pot" reaction.

In some embodiments, products of ligation (and optional second strand synthesis and/or amplification) are distributed to multiple wells and contacted with different Cas systems characterized by collateral activity activated by recognition of one of the target nucleic acids. As shown, in some embodiments, a Cas system may be or comprise a Cas12/Cas12-like (i.e., a Cas with collateral activity activated by recognition of a particular DNA sequence) system; in some embodiments, a Cas system may be or comprise a Cas13/Cas13-like (i.e., a Cas with collateral activity activated by recognition of a particular RNA sequence) system.

In some embodiments, ligation and/or extension/amplification may be performed in a "single pot". In some embodiments, extension/amplification and/or collateral cleavage may be performed in a "single pot". In some cases, ligation and/or collateral cleavage, and optionally any strand extension and/or amplification may be performed in a "single pot". Those skilled in the art will appreciate that, particularly for assays in which extension/amplification and collateral cleavage are performed in a "single pot", it may be desirable to utilize enzymes (e.g., DNA polymerase and/or Cas enzyme(s) whose relevant activity(ies) is/are sufficiently thermostable.

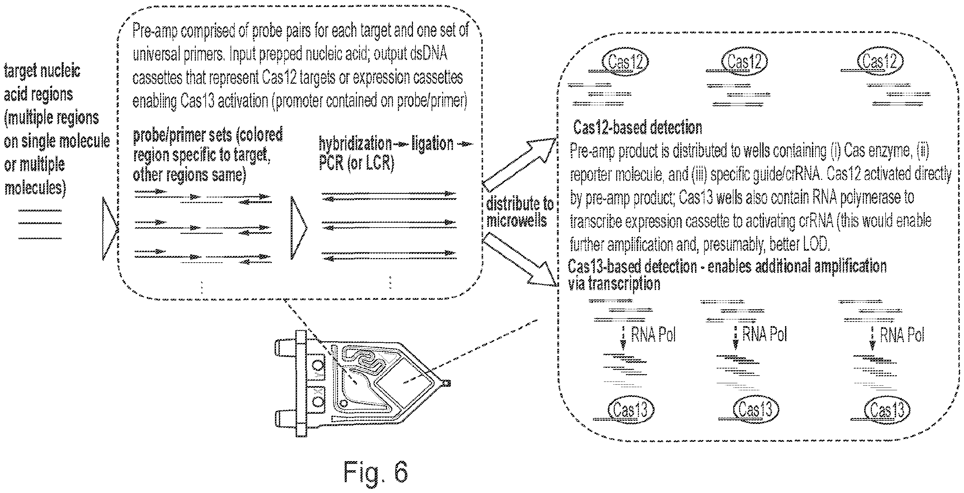

Figure 6 depicts a particular embodiment of such an exemplified assay, in which amplification is performed, and moreover all ligated products are made double stranded and then amplified by extension of a single set of "universal" primers; product is then distributed to wells, and Cas12 and/or Cas13 assays are performed. The particular embodiment illustrated by this Figure is shown to be performed in a so-called "honeycomb" tube such has been described, for example, by Cepheid, including in US patent Application US2014/0087958.

Particular advantages of an assay as depicted in Figures 5 and 6 include, for example, (i) that low copy detection can be achieved, particularly in embodiments that utilize pre-amp PCR or LCR, (transcription); (ii) high specificity - single nucleotide discrimination can be achieved on both pre-amp and Cas-activation steps; (iii) highly-multiplexable PCR/LCR with universal primers; (iv) compatible with known systems (e.g., honeycomb systems).

### Example 3 Additional Exemplary Systems with Varying Cas Recognition Element Placement

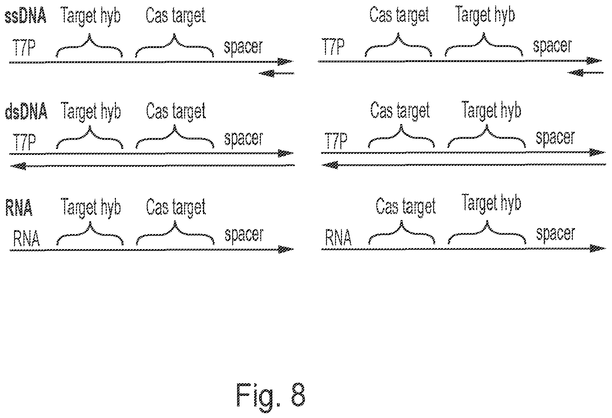

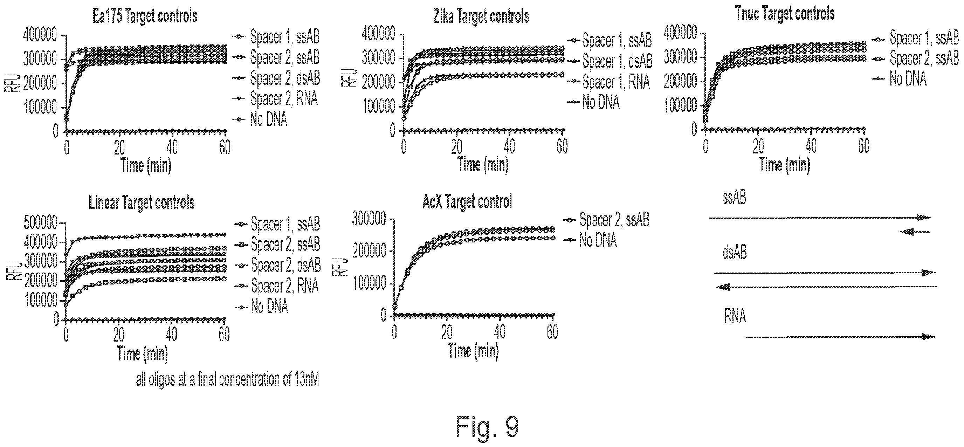

Single molecule oligonucleotides were designed to represent an oligonucleotide set as described herein after ligation (e.g., oligonucleotide A and oligonucleotide B ligated to form oligonucleotide AB) in which the Cas recognition element was either placed upstream of a target recognition element or downstream of a target recognition element in combination with additional spacer elements. See Figure 8. Each oligonucleotide was then used to produce a single Cas target RNA oligonucleotide from either RNA, dsDNA transcribed by RNA polymerase, or ssDNA converted to dsDNA *in* situ then transcribed to RNA. Figure 9 demonstrates that each oligonucleotide Cas target was able to efficiently activate Cas collateral cleavage.

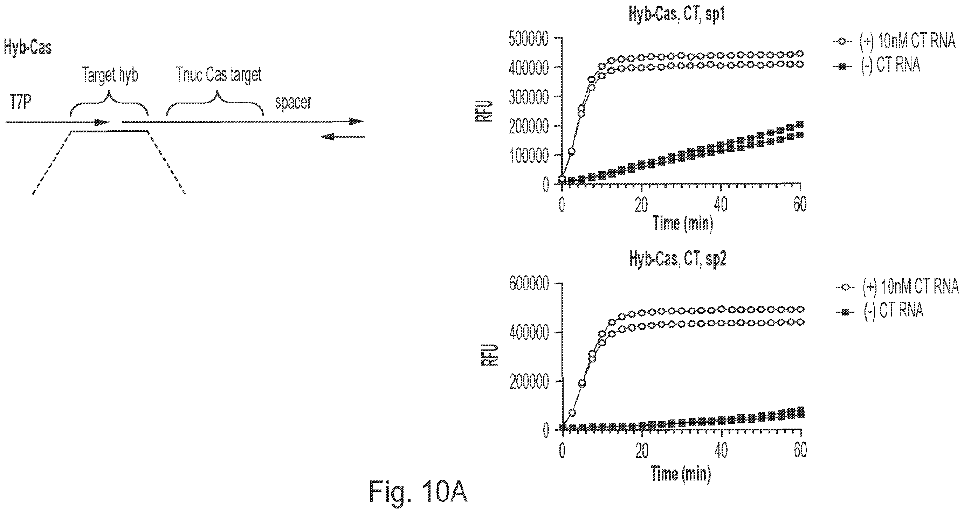

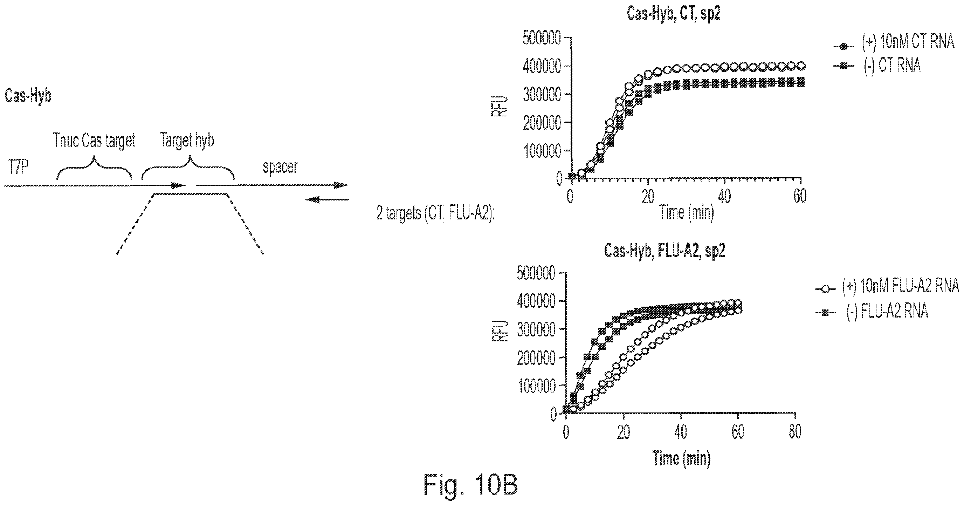

Ligation oligonucleotide sets were then designed, and incubated with a cognate target nucleic acid (CT= Chlamydia, FLU= influenza A) to perform assays as described in Figure 2. As shown in Figure 10Awhen the ligation oligonucleotide sets were designed such that Cas recognition element was downstream, 3', of the target recognition element (i.e., Cas recognition element is downstream of the hybridization element) the system was active and sensitive with low background. However, Figure 10Bdemonstrates that in the reverse order, i.e., Cas recognition element upstream, or 5', of the target recognition element the assay is not as robust and demonstrates higher background signal.

### Example 4 Demonstration of ssDNA as a Cas13 activating nucleic acid

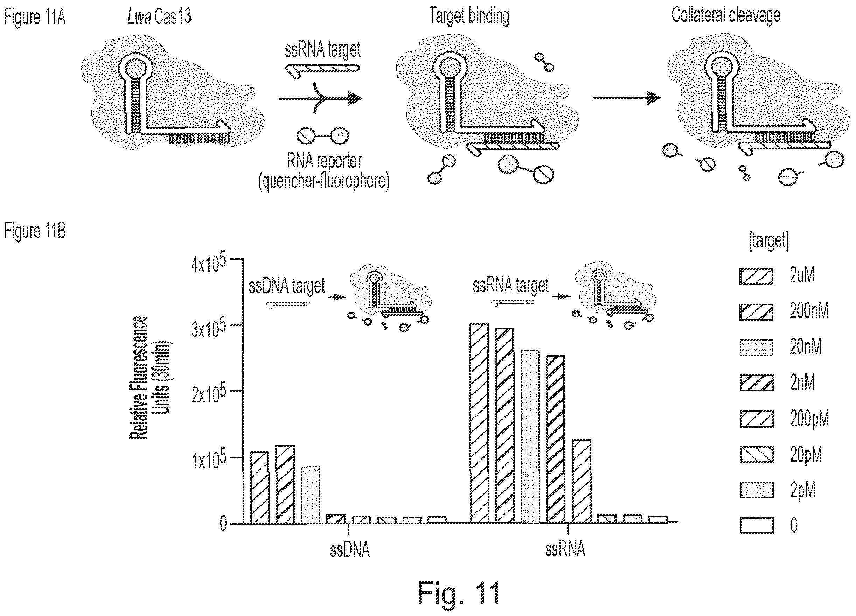

The present example demonstrates activation of *Lwa* Cas 13 activity by single-stranded DNA (ssDNA). Figure 11A shows the canonical activity of ssRNA-target Cas13 systems. Binding of a target RNA to the Cas13-guide RNA complex activates collateral Cas cleavage, which can be monitored by (for example) cleave of a fluorophore-quencher pair linked by an RNA bridge. Notably, collateral cleavage activity is observed in the presence of either a canonical ssRNA (Figure 11B, right) or ssDNA (Figure 11B, left) target sequence.

## Claims

1. A system for target specific templating of a Cas recognition element comprising:
a plurality of nucleic acid molecules having different nucleotide sequences;
a set of ligation oligonucleotides, comprising:
a first ligation oligonucleotide whose nucleotide sequence includes a templating element and a first target hybridization element, wherein the templating element i) is or comprises a promoter or complement thereof; ii) is or comprises an origin of replication or complement thereof; iii) is or comprises a binding site for a first extendable primer; or iv) is or comprises one or more transcriptional regulatory elements; and
a second ligation oligonucleotide whose nucleotide sequence includes a second target hybridization element and a Cas recognition element, wherein the Cas recognition element is a nucleotide sequence or complement thereof that is bound by a Cas guide RNA; and
a guide RNA capable of binding to said Cas recognition element,
optionally one or more bridging oligonucleotides whose nucleotide sequence is or comprises one or more additional target hybridization elements,
wherein the target hybridization elements bind to different portions of a common target site, so that, when the plurality of nucleic acid molecules includes at least one target nucleic acid molecule whose nucleotide sequence includes the target site, then the set of ligation oligonucleotides hybridizes to the target site and forms a gapped nucleic acid strand susceptible to ligation with a ligase to generate a ligated strand.

2. The system of claim 1, further comprising a ligase.

3. The system of claim 1, wherein one or more of the oligonucleotides is associated with a solid support or wherein nucleic acids of the plurality are associated with a solid support.

4. The system of claim 1, further comprising a second set of ligation oligonucleotides directed to a second target site, different from that of the first set of ligation oligonucleotides.

5. The system of claim 4, wherein the templating elements of the first and second sets of ligation oligonucleotides are the same or wherein the templating elements of the first and second sets of ligation oligonucleotides are different.

6. The system of claim 4 or 5, wherein the Cas recognition elements of the first and second sets of ligation oligonucleotides are the same or wherein the Cas recognition elements of the first and second sets of ligation oligonucleotides are different.

7. The system of any one of the prior claims iii), further comprising a first extendable primer and optionally a DNA polymerase.

8. The system of any one of the prior claims iii), wherein the second ligation oligonucleotide has a sequence that includes the complement of a binding site for a second extendable primer.

9. The system of claim 8, further comprising the second extendable primer and optionally a DNA polymerase, preferably wherein the DNA polymerase is thermostable.

10. The system of any one of the prior claims, further comprising an RNA polymerase.

11. A set of ligation oligonucleotides, comprising:
a first ligation oligonucleotide whose nucleotide sequence includes an templating element and a first target hybridization element, wherein the templating element i) is or comprises a promoter or complement thereof; ii) is or comprises an origin of replication or complement thereof; iii) is or comprises a binding site for a first extendable primer; or iv) is or comprises one or more transcriptional regulatory elements; and
a second ligation oligonucleotide whose nucleotide sequence includes a second target hybridization element and a Cas recognition element, wherein the Cas recognition element is a nucleotide sequence or complement thereof that is bound by a Cas guide RNA; and
a guide RNA capable of binding to said Cas recognition element,
optionally one or more bridging oligonucleotides whose nucleotide sequence is or comprises one or more additional target hybridization elements,
wherein the target hybridization elements bind to different portions of a common target site, so that, when the set of ligation oligonucleotides is contacted with a target nucleic acid molecule whose nucleotide sequence includes the target site, then the set of ligation oligonucleotides hybridizes to the target site and forms a gapped nucleic acid strand susceptible to ligation with a ligase to generate a ligated strand.

12. A method comprising steps of:
contacting the set of ligation oligonucleotides of claim 11 with a nucleic acid sample under conditions that permit simultaneous hybridization of the oligonucleotides in the set of ligation oligonucleotides to one or more individual nucleic acid molecules in the nucleic acid sample;
simultaneously or subsequently contacting the set of ligation oligonucleotides with ligase so that the ligated strand is generated if the nucleic acid sample includes at least one nucleic acid molecule whose nucleotide sequence includes the target site;
optionally wherein i) the templating element is or comprises a binding site for an extendible primer and the method further comprises steps of:
hybridizing the extendible primer to the ligated strand; and
extending the extendible primer so that a duplex is formed;
or ii) the templating element further is or comprises a promoter and the method comprises a step of:
transcribing from the promoter so that a transcript comprising the Cas recognition element is generated.

13. The method of claim 12, further comprising a step of duplicating one or both strands of the duplex, so that a population of single or double-stranded nucleic acid molecules, each including the Cas recognition element, is generated.

14. The method of claim 12 i) or ii), further comprising a step of contacting the Cas recognition element with:
a Cas enzyme **characterized by** collateral activity; and
a guide RNA that binds to the Cas recognition elements,
the contacting being performed while a nucleic acid probe susceptible to collateral cleavage by the Cas enzyme in present; and
detecting cleavage of the nucleic acid probe,
optionally wherein the detecting indicates that a target nucleic acid, whose nucleotide sequence includes the target site, was present in the nucleic acid sample, and the method further comprises:
quantifying the target nucleic amount present in the nucleic acid sample.

## Patentansprüche

1. System zum zielspezifischen Templating eines Cas-Erkennungselements, umfassend: eine Vielzahl von Nukleinsäuremolekülen, die unterschiedliche Nukleotidsequenzen aufweisen;
einen Satz von Ligationsoligonukleotiden, umfassend:
ein erstes Ligationsoligonukleotid, dessen Nukleotidsequenz ein Templating-Element und ein erstes Zielhybridisierungselement beinhaltet, wobei das Templating-Element i) ein Promotor oder ein Komplement davon ist oder umfasst; ii) ein Replikationsursprung oder ein Komplement davon ist oder umfasst; iii) eine Bindungsstelle für einen ersten erweiterbaren Primer ist oder umfasst; oder iv) ein oder mehrere transkriptionelle regulatorische Elemente ist oder umfasst; und
ein zweites Ligationsoligonukleotid, dessen Nukleotidsequenz ein zweites Zielhybridisierungselement und ein Cas-Erkennungselement beinhaltet, wobei das Cas-Erkennungselement eine Nukleotidsequenz oder ein Komplement davon ist, die/das von einer Cas-Guide-RNA gebunden ist; und
eine Guide-RNA, die in der Lage ist, an das Cas-Erkennungselement zu binden,
optional ein oder mehrere überbrückende Oligonukleotide, deren Nukleotidsequenz ein oder mehrere zusätzliche Zielhybridisierungselemente sind oder umfassen,
wobei die Ziel-Hybridisierungselemente an verschiedene Abschnitte einer gemeinsamen Zielstelle binden, sodass, wenn die Vielzahl von Nukleinsäuremolekülen mindestens ein ZielNukleinsäuremolekül beinhaltet, dessen Nukleotidsequenz die Zielstelle enthält, der Satz von Ligationsoligonukleotiden an die Zielstelle hybridisiert und einen Nukleinsäurestrang mit Lücken bildet, der für eine Ligation mit einer Ligase anfällig ist, um einen ligierten Strang zu erzeugen.

2. System nach Anspruch 1, ferner umfassend eine Ligase.

3. System nach Anspruch 1, wobei eines oder mehrere der Oligonukleotide mit einem festen Träger assoziiert sind oder wobei die Nukleinsäuren der Vielzahl mit einem festen Träger assoziiert sind.

4. System nach Anspruch 1, ferner umfassend einen zweiten Satz von Ligationsoligonukleotiden, der auf eine zweite Zielstelle gerichtet ist, die sich von der des ersten Satzes von Ligationsoligonukleotiden unterscheidet.

5. System nach Anspruch 4, wobei die Templating-Elemente des ersten und des zweiten Satzes von Ligationsoligonukleotiden gleich sind oder wobei die Templating-Elemente des ersten und des zweiten Satzes von Ligationsoligonukleotiden verschieden sind.

6. System nach Anspruch 4 oder 5, wobei die Cas-Erkennungselemente des ersten und des zweiten Satzes von Ligationsoligonukleotiden gleich sind oder wobei die Cas-Erkennungselemente des ersten und des zweiten Satzes von Ligationsoligonukleotiden verschieden sind.

7. System nach einem der vorherigen Ansprüche iii), ferner umfassend einen ersten erweiterbaren Primer und optional eine DNA-Polymerase.

8. Das System nach einem der vorherigen Ansprüche iii), wobei das zweite Ligationsoligonukleotid eine Sequenz aufweist, die das Komplement einer Bindungsstelle für einen zweiten erweiterbaren Primer beinhaltet.

9. System nach Anspruch 8, ferner umfassend den zweiten erweiterbaren Primer und optional eine DNA-Polymerase, wobei die DNA-Polymerase vorzugsweise thermostabil ist.

10. System nach einem der vorherigen Ansprüche, ferner umfassend eine RNA-Polymerase.

11. Satz von Ligationsoligonukleotiden, umfassend:
ein erstes Ligationsoligonukleotid, dessen Nukleotidsequenz ein Templating-Element und ein erstes Zielhybridisierungselement beinhaltet, wobei das Templating-Element i) ein Promotor oder ein Komplement davon ist oder umfasst; ii) ein Replikationsursprung oder ein Komplement davon ist oder umfasst; iii) eine Bindungsstelle für einen ersten erweiterbaren Primer ist oder umfasst; oder iv) ein oder mehrere transkriptionelle regulatorische Elemente ist oder umfasst; und
ein zweites Ligationsoligonukleotid, dessen Nukleotidsequenz ein zweites Zielhybridisierungselement und ein Cas-Erkennungselement beinhaltet, wobei das Cas-Erkennungselement eine Nukleotidsequenz oder ein Komplement davon ist, die/das von einer Cas-Guide-RNA gebunden ist; und
eine Guide-RNA, die in der Lage ist, an das Cas-Erkennungselement zu binden, optional ein oder mehrere überbrückende Oligonukleotide, deren Nukleotidsequenz ein oder mehrere zusätzliche Zielhybridisierungselemente sind oder umfassen,
wobei die Zielhybridisierungselemente an verschiedene Abschnitte einer gemeinsamen Zielstelle binden, sodass, wenn der Satz von Ligationsoligonukleotiden mit einem Zielnukleinsäuremolekül in Kontakt gebracht wird, dessen Nukleotidsequenz die Zielstelle beinhaltet, der Satz von Ligationsoligonukleotiden an die Zielstelle hybridisiert und einen Nukleinsäurestrang mit Lücken bildet, der für eine Ligation mit einer Ligase anfällig ist, um einen ligierten Strang zu erzeugen.

12. Verfahren, umfassend die folgenden Schritte:
Inkontaktbringen des Satzes von Ligationsoligonukleotiden nach Anspruch 11 mit einer Nukleinsäureprobe unter Bedingungen, die eine gleichzeitige Hybridisierung der Oligonukleotide in dem Satz von Ligationsoligonukleotiden mit einem oder mehreren einzelnen Nukleinsäuremolekülen in der Nukleinsäureprobe erlauben;
gleichzeitiges oder aufeinanderfolgendes Inkontaktbringen des Satzes von Ligationsoligonukleotiden mit Ligase, sodass der ligierte Strang erzeugt wird, wenn die Nukleinsäureprobe mindestens ein Nukleinsäuremolekül beinhaltet, dessen Nukleotidsequenz die Zielstelle beinhaltet;
optional wobei i) das Templating-Element eine Bindungsstelle für einen erweiterbaren Primer ist oder umfasst und das Verfahren ferner folgende Schritte umfasst:
Hybridisieren des erweiterbaren Primers an den ligierten Strang; und
Erweitern des erweiterbaren Primers, sodass ein Duplex gebildet wird;
oder ii) das Templating-Element ferner ein Promotor ist oder umfasst und das Verfahren einen folgenden Schritt umfasst:
Transkribieren von dem Promotor, sodass ein Transkript erzeugt wird, das das Cas-Erkennungselement umfasst.

13. Verfahren nach Anspruch 12, ferner umfassend einen Schritt eines Duplizierens eines oder beider Stränge des Duplexes, sodass eine Population von einzel- oder doppelsträngigen Nukleinsäuremolekülen erzeugt wird, die jeweils das Cas-Erkennungselement beinhalten.

14. Verfahren nach Anspruch 12 i) oder ii), das ferner umfassend einen Schritt eines Inkontaktbringens des Cas-Erkennungselements mit:
einem Cas-Enzym, das durch kollaterale Aktivität gekennzeichnet ist; und
eine Guide-RNA, die an die Cas-Erkennungselemente bindet, wobei das Inkontaktbringen ausgeführt wird, während eine Nukleinsäuresonde vorhanden ist, die für eine kollaterale Spaltung durch das Cas-Enzym anfällig ist; und
Detektieren einer Spaltung der Nukleinsäuresonde,
optional wobei das Detektieren angibt, dass eine Zielnukleinsäure, deren Nukleotidsequenz die Zielstelle beinhaltet, in der Nukleinsäureprobe vorhanden war, und das Verfahren ferner Folgendes umfasst:
Quantifizieren der in der Nukleinsäureprobe vorhandenen Nukleinmenge.

## Revendications

1. Système pour la formation de matrice spécifique d'un élément de reconnaissance Cas comprenant : une pluralité de molécules d'acides nucléiques ayant différentes séquences nucléotidiques;
un ensemble d'oligonucléotides de ligature, comprenant :
un premier oligonucléotide de ligature dont la séquence nucléotidique comprend un élément servant de matrice et un premier élément d'hybridation cible, dans lequel l'élément servant de matrice i) est ou comprend un promoteur ou un complément de celui-ci ; ii) est ou comprend une origine de réplication ou un complément de celle-ci ; iii) est ou comprend un site de liaison pour une première amorce extensible ; ou iv) est ou comprend un ou plusieurs éléments régulateurs transcriptionnels ; et
un second oligonucléotide de ligature dont la séquence nucléotidique comprend un second élément d'hybridation cible et un élément de reconnaissance Cas, dans lequel l'élément de reconnaissance Cas est une séquence nucléotidique ou un complément de celle-ci qui est lié par un ARN guide Cas ; et
un ARN guide capable de se lier audit élément de reconnaissance Cas,
éventuellement un ou plusieurs oligonucléotides de pont dont la séquence nucléotidique est ou comprend un ou plusieurs éléments d'hybridation cibles supplémentaires,
les éléments d'hybridation cibles se liant à différentes parties d'un site cible commun, de sorte que, lorsque la pluralité de molécules d'acides nucléiques comprend au moins une molécule d'acide nucléique cible dont la séquence nucléotidique comprend le site cible, puis l'ensemble des oligonucléotides de ligature s'hybride au site cible et forme un brin d'acide nucléique discontinu susceptible de ligature avec une ligase pour produire un brin ligaturé.

2. Système de la revendication 1, comprenant en outre une ligase.

3. Système de la revendication 1, dans lequel un ou plusieurs oligonucléotides sont associés avec un support solide ou dans lequel les acides nucléiques de la pluralité sont associés à un support solide.

4. Système de la revendication 1, comprenant en outre un second ensemble d'oligonucléotides de ligature dirigés vers un second site cible, différent de celui du premier ensemble d'oligonucléotides de ligature.

5. Système de la revendication 4, dans lequel les éléments servant de matrice du premier et du second ensemble d'oligonucléotides de ligature sont les mêmes ou dans lequel les éléments servant de matrice Cas du premier et du second ensemble d'oligonucléotides de ligature sont différents.

6. Système de la revendication 4 ou 5, dans lequel les éléments de reconnaissance Cas du premier et du second ensemble d'oligonucléotides de ligature sont les mêmes ou dans lequel les éléments de reconnaissance Cas du premier et du second ensemble d'oligonucléotides de ligature sont différents.

7. Système de l'une quelconque des revendications précédentes iii), comprenant en outre une première amorce extensible et éventuellement une ADN polymérase.

8. Système de l'une quelconque des revendications précédentes iii), dans lequel le second oligonucléotide de ligature a une séquence qui comprend le complément d'un site de liaison pour une seconde amorce extensible.

9. Système de la revendication 8, comprenant en outre la seconde amorce extensible et éventuellement une ADN polymérase, de préférence dans lequel l'ADN polymérase est thermostable.

10. Système de l'une quelconque des revendications précédentes, comprenant en outre une ARN polymérase.

11. Ensemble d'oligonucléotides de ligature, comprenant :
un premier oligonucléotide de ligature dont la séquence nucléotidique comprend un élément servant de matrice et un premier élément d'hybridation cible, dans lequel l'élément servant de matrice i) est ou comprend un promoteur ou un complément de celui-ci ; ii) est ou comprend une origine de réplication ou un complément de celle-ci ; iii) est ou comprend un site de liaison pour une première amorce extensible ; ou iv) est ou comprend un ou plusieurs éléments régulateurs transcriptionnels ; et
un second oligonucléotide de ligature dont la séquence nucléotidique comprend un second élément d'hybridation cible et un élément de reconnaissance Cas, l'élément de reconnaissance Cas étant une séquence nucléotidique ou un complément de celle-ci qui est lié par un ARN guide Cas ; et
un ARN guide capable de se lier audit élément de reconnaissance Cas,
éventuellement un ou plusieurs oligonucléotides de pont dont la séquence nucléotidique est ou comprend un ou plusieurs éléments d'hybridation cibles supplémentaires,
les éléments d'hybridation cible se liant à différentes parties d'un site cible commun, de sorte que, lorsque l'ensemble des oligonucléotides de ligature est en contact avec une molécule d'acide nucléique cible dont la séquence nucléotidique inclut le site cible, puis l'ensemble des oligonucléotides de ligature s'hybride au site cible et forme un brin d'acide nucléique discontinu susceptible de la ligature avec une ligase pour produire un brin ligaturé.

12. Procédé comprenant les étapes de :
mise en contact de l'ensemble des oligonucléotides de ligature de la revendication 11 et d'un échantillon d'acide nucléique dans des conditions qui permettent l'hybridation simultanée des oligonucléotides dans l'ensemble des oligonucléotides de ligature avec une ou plusieurs molécules d'acide nucléique individuelles dans l'échantillon d'acide nucléique ;
simultanément ou subséquemment mise en contact de l'ensemble des oligonucléotides de ligature avec la ligase de sorte que le brin ligaturé est produit si l'échantillon d'acide nucléique comprend au moins une molécule d'acide nucléique dont la séquence nucléotidique comprend le site cible ;
éventuellement dans lequel i) l'élément servant de matrice est ou comprend un site de liaison pour une amorce extensible et le procédé comprend en outre des étapes de :
hybridation de l'amorce extensible au brin ligaturé ; et
extension de l'amorce extensible de sorte qu'un duplex est formé ;
ou ii) l'élément servant de matrice est ou comprend un promoteur et le procédé comprend une étape de :
transcription à partir du promoteur de manière à ce qu'une transcription comprenant l'élément de reconnaissance Cas soit produite.

13. Procédé de la revendication 12, comprenant en outre une étape de duplication d'un ou des deux brins du duplex, de sorte qu'une population de molécules d'acide nucléique simple ou double brin, chacune comprenant l'élément de reconnaissance Cas, est produite.

14. Procédé de la revendication 12 i) ou ii), comprenant en outre une étape de mise en contact de l'élément de reconnaissance Cas avec :
une enzyme Cas **caractérisée par** une activité collatérale ; et
un ARN guide qui se lie aux éléments de reconnaissance Cas, la mise en contact étant effectué alors qu'une sonde d'acide nucléique sensible au clivage collatéral par l'enzyme Cas est présente ; et
la détection du clivage de la sonde d'acide nucléique,
éventuellement, lorsque la détection indique qu'un acide nucléique cible, dont la séquence nucléotidique comprend le site cible, était présent dans l'échantillon d'acide nucléique, et le procédé comprenant :
la quantification de la quantité d'acide nucléique cible présente dans l'échantillon d'acide nucléique.
